# EUROPEAN PATENT APPLICATION

(11) **EP 1 170 027 A1**
(43) Date of publication of application: **09.01.2002**
(21) Application number: 01305573.6
(22) Date of filing: 27.06.2001
(51) Int. Cl.: A61M 25/06

(54) **A method for vascular access devices**

(30) Priority: 28.06.2000 US 605772
(71) Applicant: ETHICON, INC., Somerville New Jersey 08876 (US)
(72) Inventor: Kafrawy, Adel, Kingston, MA 02364 (US); Onwumere, Fidelis, Mansfield, TX 76063 (US); Kovalic, Gerald, Southlake, TX 76092 (US); Solomon, Donald, Southlake, TX 76092 (US)
(74) Representative: Mercer, Christopher Paul

(57) **Abstract**

A catheter device comprises a needle and a head to the needle formed as one-piece. The one-piece needle and the head to the needle is inserted into a one-piece catheter and hub.

## Description

### Field of the Invention

This invention relates generally to a method of forming an intravascular device and more specifically for assembling a catheter device.

### Description of Related Art

Intravascular devices such as catheter assemblies are generally used for passing fluids between a device such as a syringe or a drip to or from body lumens such as veins or arteries, or other internal target sites. Such an assembly usually includes a hub, a catheter tube, and a needle. An eyelet ring is typically inserted into the catheter tube. The catheter tube, together with the eyelet ring, is then inserted into an opening in the nose of the hub and is secured to the hub by press fitting the eyelet ring within the nose of the hub. This hub and tube assembly is then mounted over a sharp needle which is in turn attached to a plastic hub. The sharp tip of the needle is used for piercing a body lumen so that access may be gained into the body lumen by the needle and subsequently the catheter. Once the catheter and the needle are located within the body lumen, the needle is removed and discarded while the catheter tube remains in the body lumen. A syringe or a tube of a drip is then attached to the hub so that fluids may be passed through the hub and the catheter between the drip or the syringe and the body lumen. The hub is typically made of materials that provide sufficient rigidity to securely attach drip lines thereto and the catheter tube is usually made of a material which is flexible and soft to minimize bodily injury.

Typically, seven or eight components comprise a catheter device such as a Peripheral Intravenous Catheter ("PIVC"). The cost to manufacture a catheter device includes the cost of manufacturing each component of the catheter device and the cost of assembling the components. It is desirable to reduce the cost of manufacturing and assembling the components of a catheter device.

### SUMMARY OF THE INVENTION

A catheter device is disclosed that comprises a one-piece needle and a head of a needle. The one-piece needle and the head of the needle is inserted into a one-piece catheter and hub. Additional features, embodiments, and benefits will be evident in view of the figures and detailed description presented herein.

### BRIEF DESCRIPTION OF THE DRAWINGS

The features, aspects, and advantages of the invention will become more thoroughly apparent from the following detailed description, appended claims, and accompanying drawings in which:
**Figure 1** illustrates a one-piece needle in accordance with one embodiment of the invention.
**Figure 2** illustrates a one-piece catheter and hub in accordance with one embodiment of the invention.
**Figure 3** illustrates a one-piece needle coaxially nestled in a one-piece catheter and hub and blood flowing from the distal end of the needle to the proximal end of the head of the needle.
**Figure 4** illustrates a plurality of one-piece needles located on a rotatable table in which each needle is automatically inserted into catheter and hub by an arm coupled to the rotatable table in accordance with one embodiment of the invention.
**Figure 5** illustrates a one-piece needle that moves along an angled-slide like device and as the one-piece needle leaves the slide, an arm secured to a table strikes the needle at the proximal end of the needle in accordance with one embodiment of the invention.
**Figure 6** illustrates inserting a one-piece needle into one-piece catheter and hub in accordance with one embodiment of the invention.
**Figure 7** illustrates a one-piece needle, plug, and spiral head in accordance with one embodiment of the invention.
**Figure 8** illustrates a two-piece catheter device in accordance with one embodiment of the invention.
**Figure 9** illustrates a one-piece spiral needle, plug, and spiral head in accordance with one embodiment of the invention.
**Figure 10** illustrates a two-piece catheter device in accordance with one embodiment of the invention.
**Figure 11** illustrates a one-piece hooked needle in accordance with one embodiment of the invention.
**Figure 12** illustrates the hooked one-piece needle inserted into a one-piece catheter and hub.
**Figure 13** illustrates a one-piece spiraled needle in accordance with one embodiment of the invention.
**Figure 14** illustrates a one-piece spiral needle inserted into a one-piece catheter and hub in accordance with one embodiment of the invention.
**Figure 15** illustrates a one-piece needle having a rectangular proximal end in accordance with one embodiment of the invention.
**Figure 16** illustrates the one-piece needle with a rectangular proximal end inserted into a one-piece catheter and hub in accordance with one embodiment of the invention.
**Figure 17** illustrates a two-piece needle, plug, and head in accordance with one embodiment of the invention.
**Figure 18** illustrates a three-piece catheter device in accordance with one embodiment of the invention.
**Figure 19** illustrates a two-piece needle, plug, and head in accordance with one embodiment of the invention.
**Figure 20** illustrates a one-piece catheter and hub in accordance with one embodiment of the invention.
**Figure 21** illustrates a three-piece catheter device in accordance with one embodiment of the invention.
**Figure 22** illustrates a larger scale view of a three-piece catheter device with vents in accordance with one embodiment of the invention.
**Figure 23** illustrates a larger scale view of a three-piece catheter device in accordance with one embodiment of the invention.
**Figure 24** illustrates a three-piece needle into a flashback chamber, and porous plug assembly in accordance with one embodiment of the invention.
**Figure 25** illustrates a four-piece catheter device in accordance with one embodiment of the invention.
**Figure 26** illustrates a one-piece catheter and hub that may be used in a catheter device in accordance with one embodiment of the invention.
**Figure 27** illustrates a one-piece catheter and hub that may be used in a catheter device in accordance with one embodiment of the invention.
**Figures 28** illustrates a one-piece catheter and hub having grooves in the hub portion in accordance with one embodiment of the invention.
**Figures 29** illustrates a one-piece catheter and hub having grooves in the hub portion in accordance with one embodiment of the invention.
**Figures 30** illustrates a one-piece catheter and hub having grooves in the hub portion in accordance with one embodiment of the invention.
**Figures 31** illustrates a one-piece catheter and hub having grooves in the hub portion in accordance with one embodiment of the invention.
**Figure 32** illustrates a one-piece catheter and hub in accordance with one embodiment of the invention.

### DETAILED DESCRIPTION OF THE INVENTION

In the description that follows, the invention is described with reference to specific embodiments thereof. It will, however, be evident that various modifications and changes may be made thereto without departing from the broader spirit and scope of the invention as set forth in the claims. The specification and drawings are, accordingly, to be regarded in an illustrative rather than a restrictive sense.

One embodiment of the invention relates to a catheter device comprising an integral one-piece needle that is inserted into a one-piece catheter and hub. The one-piece needle comprises a head portion and a needle portion. The catheter and hub may be beveled. There are several advantages to assembling a one-piece needle to a one-piece catheter and hub to form a catheter device. For example, less components are separately manufactured and later assembled. Moreover, the quality of the catheter device is improved because the precise dimensions of each single piece is manufactured at one time.
It will be appreciated that the one-piece, two-piece, three-piece, and four-piece needle and head or a needle, plug, and head are manufactured by techniques described in the following co-pending European patent applications:
EP-A- , claiming priority from USSN 09/608673, for a Single-Piece Needle (Attorney's ref: PO27437), and
EP-A- , claiming priority from USSN 09/608671, for a One-Piece Needle (Attorney's ref: P027438). Additionally, the needle, head, and plug may be comprised of a variety of materials such as the compounds described in the above-referenced "Single-Piece Needle" application. Alternatively, the needle, head, and plug may also comprise a metal such as stainless steel. In this case, the needle, head, and plug are formed using a molten metal instead of a molten polymer as described in the above-referenced "One-Piece Needle" application.

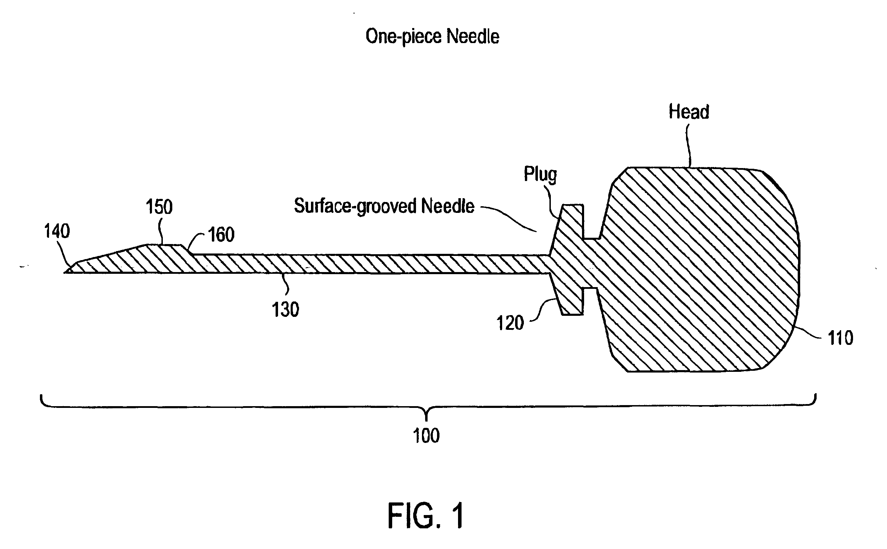

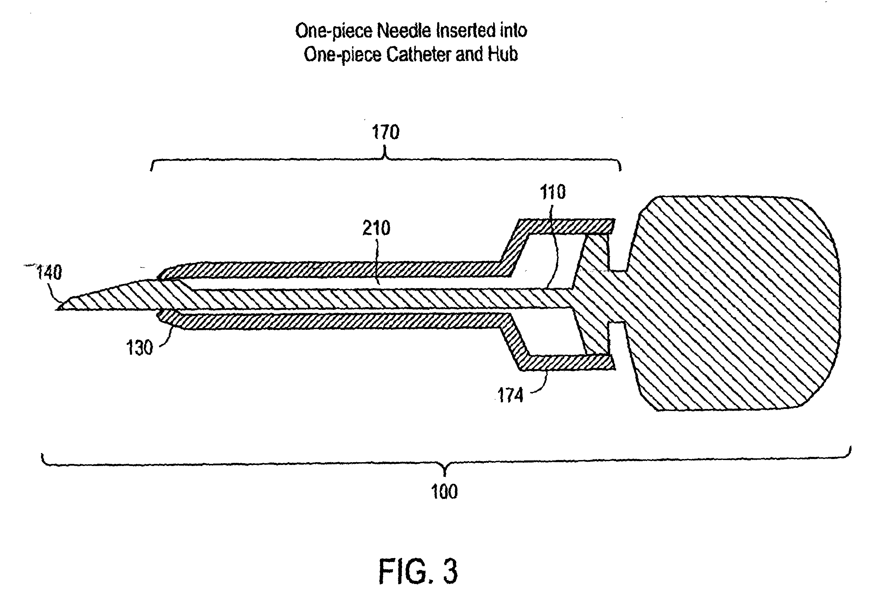

**Figures 1-3** represent a two-piece catheter device in accordance with one embodiment of the invention. **Figure 1** illustrates a one-piece surface grooved needle 100. One-piece needle 100 includes needle portion 130, plug 120, and head 110. Needle portion 130 has a beveled distal tip 140 that transitions to a flat surface 150 and angled portion 160. Angled portion 160 thereafter transitions to a flat surface. At the proximal end of needle portion 130 is plug 120. Plug 120 fits securely inside one-piece catheter and hub 170 shown in **Figure 3** and generally prevents blood or bodily fluids from spilling out of the hub portion 174. One purpose of head 110 is to provide a healthcare worker with an object to grip between his or her fingers before, during, or after the catheter device has been inserted into the patient.

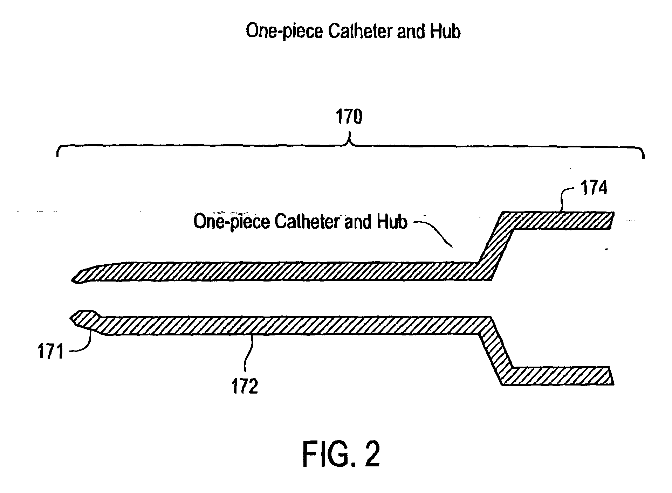

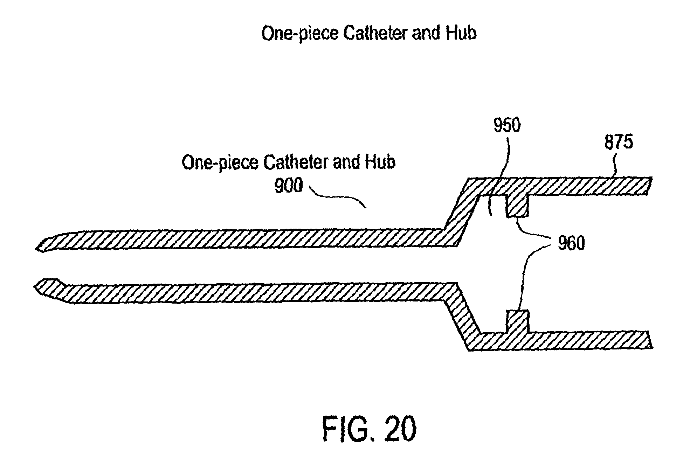

Figure 2 illustrates a one-piece catheter and hub 170 that is formed by a process described in EP-A-1 116 567. The process describes a method and an apparatus for forming a one-piece catheter and hub. It will be appreciated that a variety of catheter and hubs may be manufactured by the process disclosed in the above-referenced "Single-Piece Needle" application. Figures 20 and 26-32 represent a few of the one-piece catheter and hubs that may be formed from the process disclosed in the above-referenced "Single-Piece Needle" application and these one-piece catheters and hubs are used to form a catheter device. One-piece catheter and hub 170 has a beveled distal tip 171, and a tub portion 172. The proximal end of the tube portion 172 then broadens and transitions into hub portion 174.

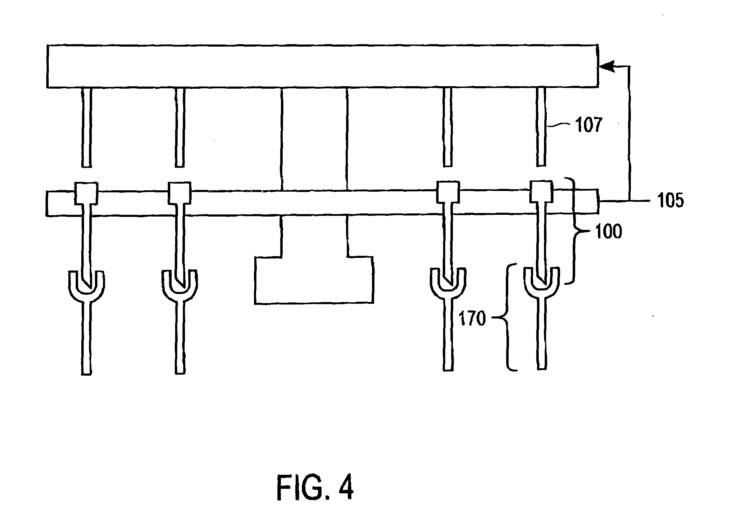

**Figure 3** illustrates a one-piece surface grooved needle 100 having a head. One-piece needle is coaxially nestled in a one-piece catheter and hub 170 and blood flowing from the distal tip 140 of needle portion 130 to the proximal end of one-piece needle 110 through channel 210. It will be appreciated that a variety of methods may be used to assemble the one-piece needle 100 with the one-piece catheter and hub. Some of these methods are described in **Figures 4-6**.

**Figure 4** illustrates a plurality of one-piece needles 100 located on a rotatable table 105 in which each one-piece needle 100 is automatically inserted into one-piece catheter and hub 170 by an arm 107 coupled to the rotatable table. It will be appreciated that a plurality of arms 107 could be secured to the rotatable table allowing the one-piece needle 100 to be automatically placed into the one-piece catheter and hub 170 after one-piece needle 100 is positioned directly over one-piece catheter and hub 170.

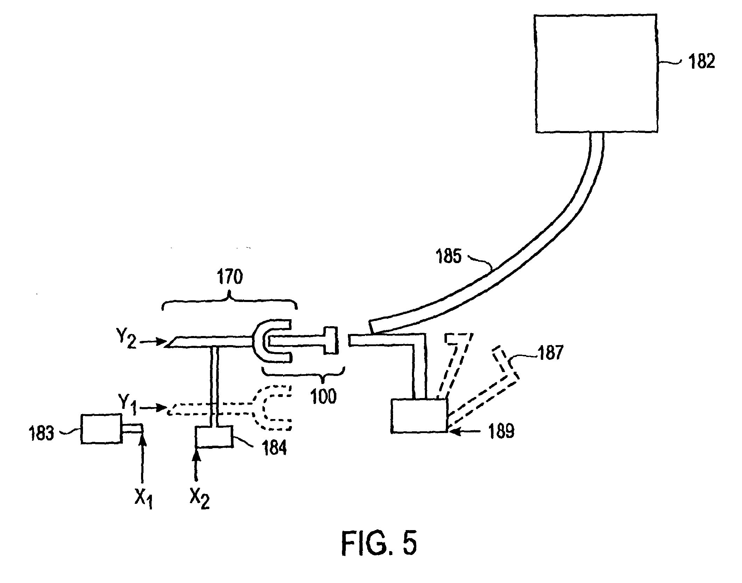

**Figure 5** illustrates another method of assembling one-piece needle 100 into one-piece catheter and hub 170. One-piece catheter and hub is pushed from position X₁ to position X₂ by positioning arm 183. One-piece catheter and hub 170 is placed into a position to receive one-piece needle 100 by securing arm 184.

Securing arm 184 automatically moves one-piece catheter and hub 170 from a first position Y₁ to a second position Y₂. At second position Y₂, one-piece catheter and hub 170 is able to receive one-piece needle 100. One-piece needle 100 moves along an angled-slide like device 185 and as the one-piece needle 100 leaves angled-slide device 185, an arm 187 secured to a table 189, strikes, hits, or taps one-piece needle 100 at the proximal end ("head") of one-piece needle 100. This causes the one-piece needle 100 to enter the one-piece catheter and hub 170.

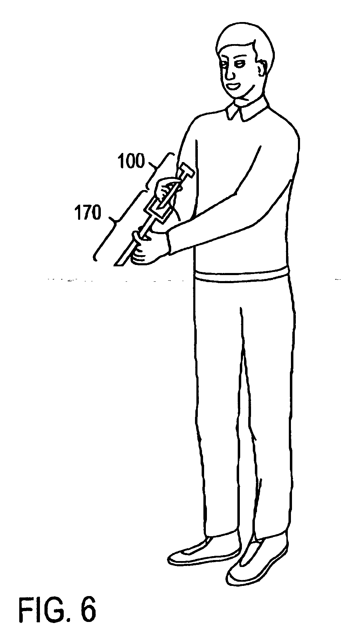

**Figure 6** illustrates another method of inserting one-piece needle and hub 100 into a one-piece catheter and hub 170. This method involves an operator simply placing one-piece needle 100 into one-piece catheter and hub 170. This method is not as efficient as other methods that automatically insert the one-piece needle 100 into one-piece catheter and hub 170.

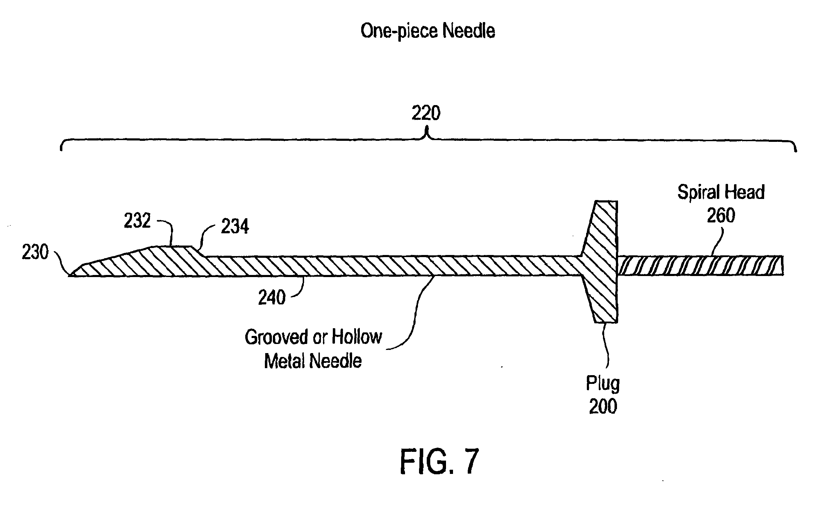

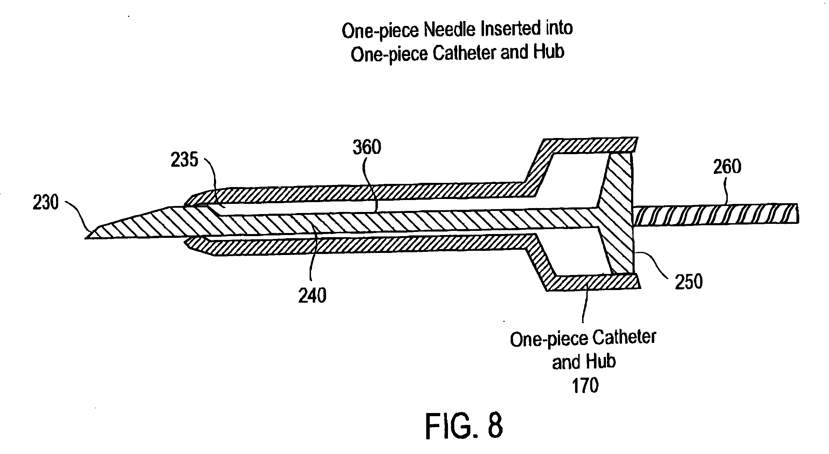

**Figure 7** illustrates a one-piece needle, plug, and spiral head 220. Needle 240 has a shaft that may be surface grooved or hollow. If needle 240 is surface grooved, it will have a distal tip 230 that transitions to flat portion 232 and thereafter to angled portion 234. Plug 250 performs the same function as described above. At the proximal end of plug 250 is spiral head 260. One purpose of spiral head 260 relates to providing a healthcare working with an element to grip while inserting or holding the catheter device into the patient. One-piece needle, plug, and spiral head 200 may comprise a polymeric compound or a metallic material using one of the methods described in the above-referenced "Single-Piece Needle" and "One-Piece Needle" applications. One-piece needle, plug, and spiral head 220 is then coaxially nestled into the one-piece catheter and hub 170 shown in Figure 8.

In reference to **Figure 7**, **Figure 8** illustrates a one-piece needle, plug, and spiral head 220 coaxially nestled into one-piece catheter and hub 170. The distal tip 230 punctures the skin and the vein, causing blood to enter the distal end of the groove 235 and the blood proceeds along the groove or channel 360. Blood enters the groove of needle 240 and travels within groove or channel 360 in the proximal direction of spiral head 260. Plug 250 generally secures the hub to the head of the needle such as spiral head 260.

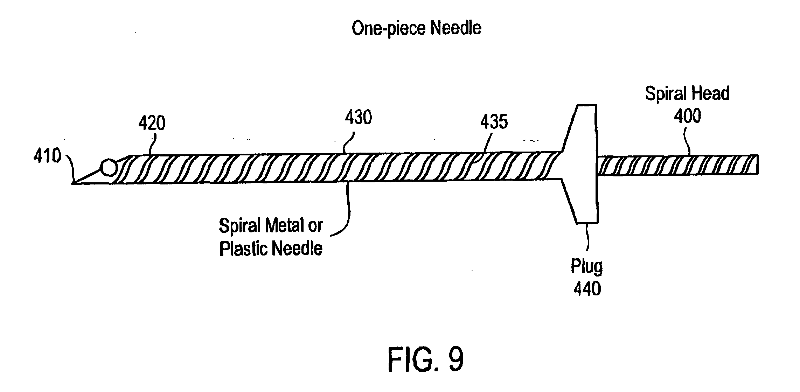

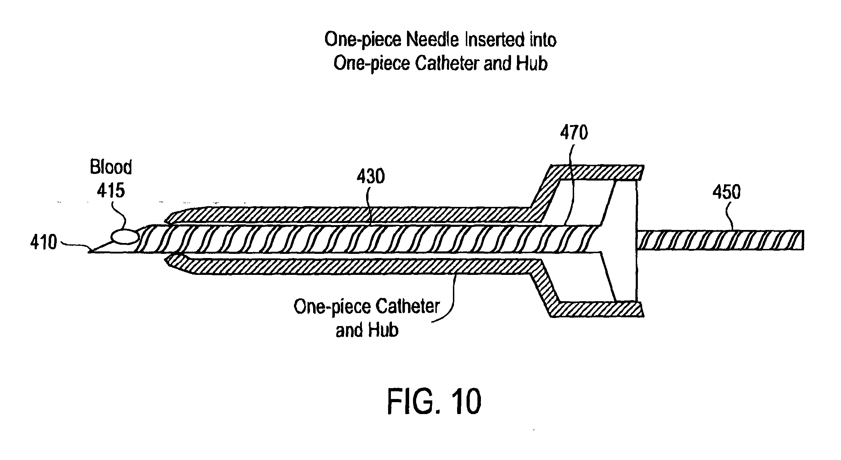

**Figures 9** and **10** illustrate the assembly of a two-piece catheter device in accordance with one embodiment of the invention. **Figure 9** illustrates one-piece metallic or polymeric spiral needle, plug, and spiral head 400. Needle 430 has a beveled distal tip 410 that transitions to a flat surface 420. Each spiral 435 that surrounds needle 430, generally has a diameter that ranges from approximately 0.05 to 0.2 inches in diameter. The distance between each spiral generally ranges from 0.02 to 0.2 inches. It will be appreciated that spirals may be replaced with helical-shaped spirals or other suitable shapes. Spirals 435 generally surround the entire length of needle 430 up to the distal end of plug 440 as illustrated in **Figure 9.**

**Figure 10** shows that at the proximal end of plug 440 is spiral head 450. Spiral head 450 allows a healthcare worker to grip the spiral head 450 while inserting or holding the catheter device in the body of the patient. After the single integral piece needle, plug, and spiral head 400 is formed, this single integral piece is then inserted into the one-piece catheter and hub as illustrated in **Figure 10** using any one of the methods described in **Figures 4-6** or any other conventional methods. **Figure 10** also illustrates blood entering beveled distal tip 410 of needle 430. The blood enters aperture 415 and travels from the distal end to the proximal end of needle 430. The blood exits needle 430 and enters hub 470.

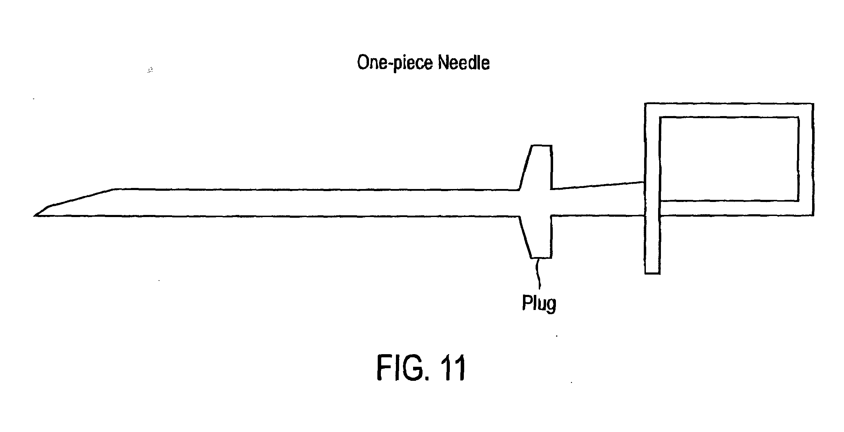

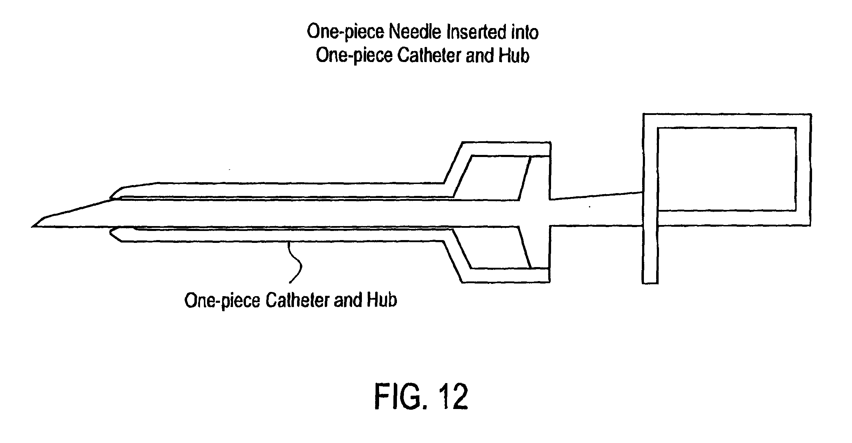

**Figure 11** illustrates a one-piece hooked needle in accordance with one embodiment of the invention. The one-piece hooked needle is inserted into the one-piece catheter and hub illustrated in **Figure 12**. It will be appreciated that the disk or flange-like portion of the one-piece hooked needle may contact the inner surface of the hub portion of the one-piece catheter and hub. In another embodiment, the disk portion of the one-piece needle may lock into place at point A. When the disk locks into place, a clicking noise is emitted.

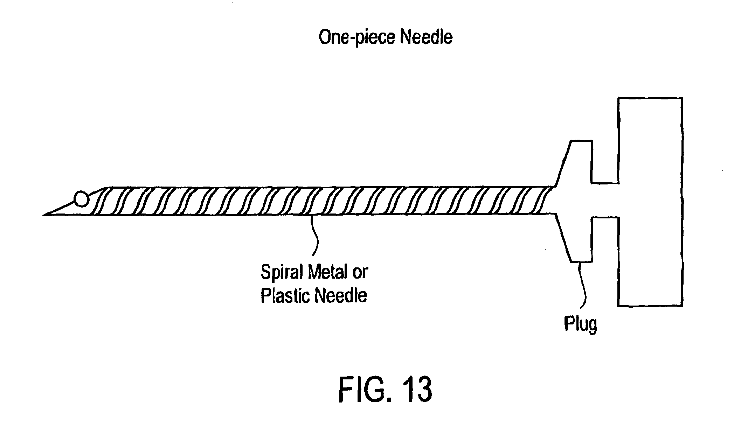

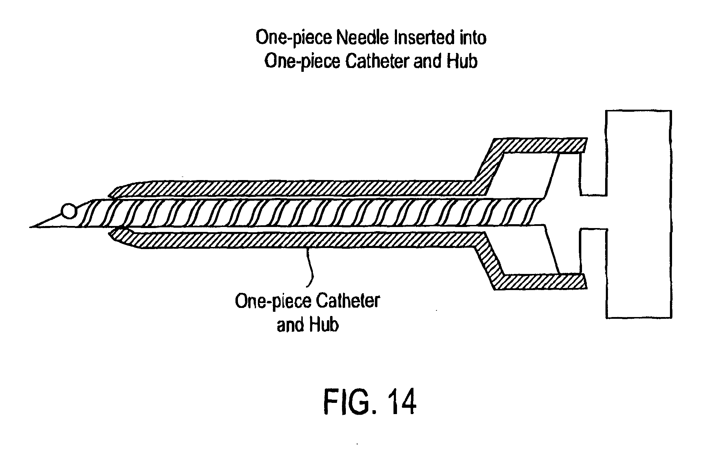

**Figures 13** and **14** illustrates a two-piece catheter device that is assembled using one of the methods described in **Figures 4-6** or any other conventional methods. **Figure 13** illustrates a one-piece spiral needle. It will be appreciated that although the spirals are shown being substantially equally distanced apart from one another on one portion of the longitudinal section of the one-piece needle, other embodiments would allow for the unequal distance between the spirals. For example, the distance from a first spiral may be 0.05 inches from a second spiral whereas the distance from the second spiral to a third spiral may be 0.1 inches. **Figure 14** illustrates the one-piece spiral needle inserted into a one-piece catheter and hub.

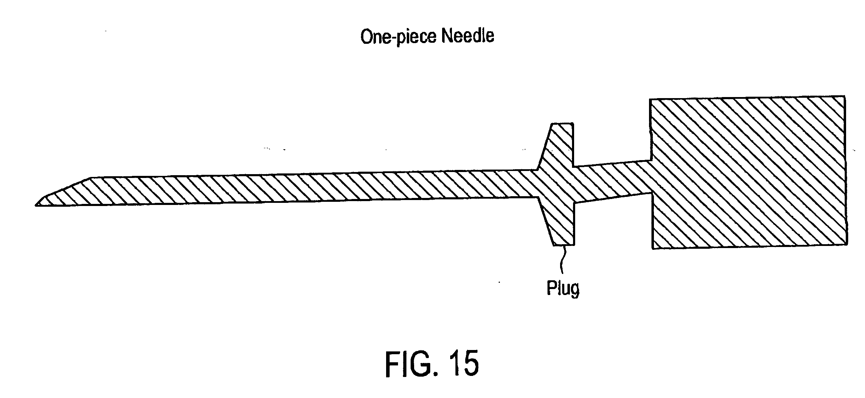

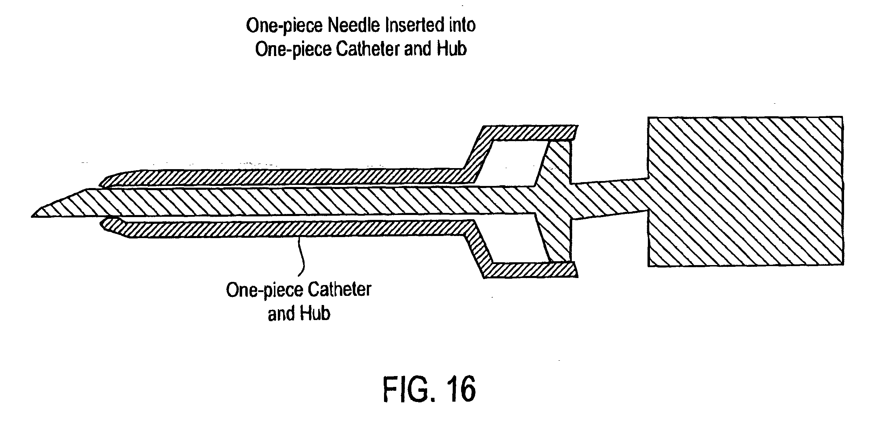

**Figures 15** and **16** illustrate another embodiment of the two-piece catheter device. **Figure 15** illustrates a one-piece needle with a substantially rectangular proximal end. The purpose of having a substantially rectangular proximal portion of the needle is to allow the one-piece needle to lock or snap into place with the one-piece catheter and hub illustrated in **Figure 16**, even in the absence of the plug.

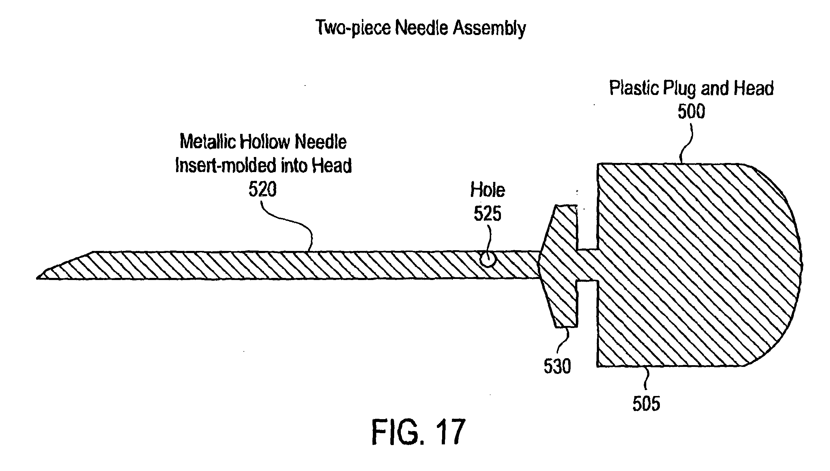

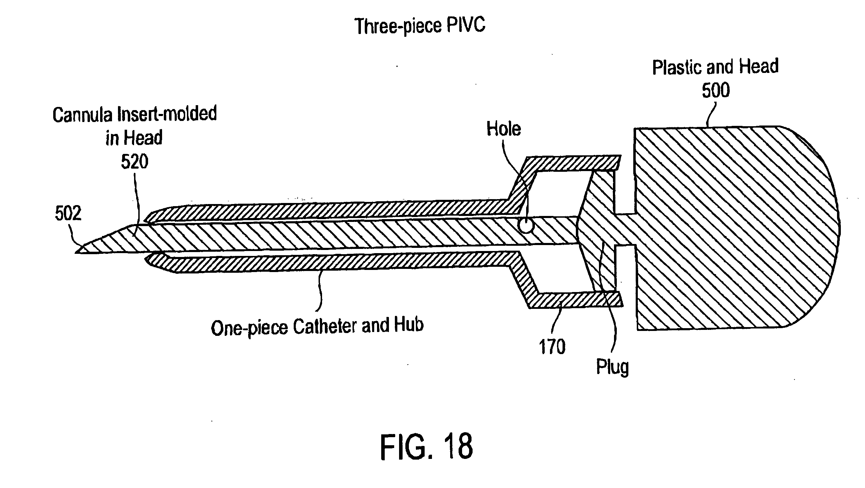

**Figures 17** and **18** illustrate three pieces assembled into a three-piece catheter device. **Figure 17** illustrates a two-piece needle, plug and head 500. Needle 520 may be grooved or hollow made from metallic or polymeric material. A hole or an aperture 525 may be located in needle 520. It will be appreciated that a hole or aperture is unnecessary for the grooved needle but is necessary for the hollow needle.

Needle 520 is coupled to plug 530 and head 505. Needle 520 is secured to plug 530 and head 505 by insert injection molding, by press-fitting, or using an ultraviolet-activated adhesive. This two-piece assembly is then coaxially nestled into the one-piece catheter and hub 170 shown in **Figure 2**.

**Figure 18** illustrates a three-piece catheter device assembled in accordance with one embodiment of the invention. Two-piece needle, plug and head 500 is coaxially nestled into one-piece catheter and hub 170 shown in **Figure 2**. Blood enters distal tip 502 of needle 520 to the distal end of plug 530 where a hole is drilled.

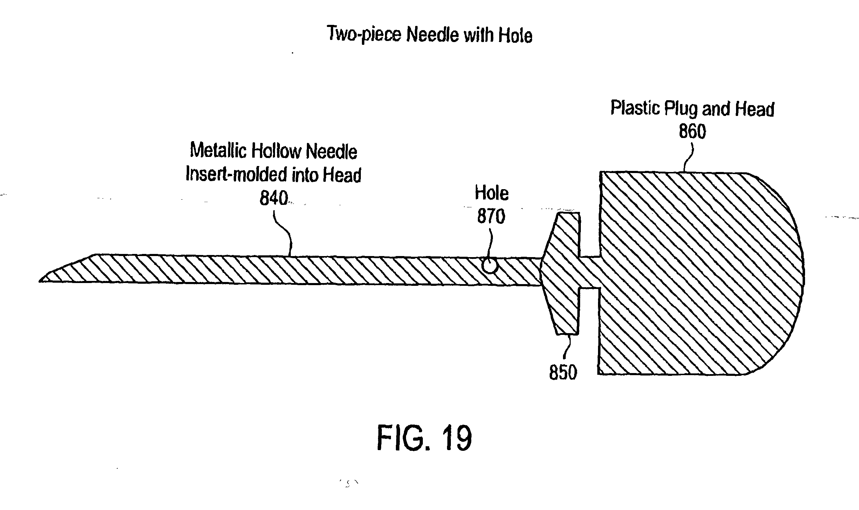

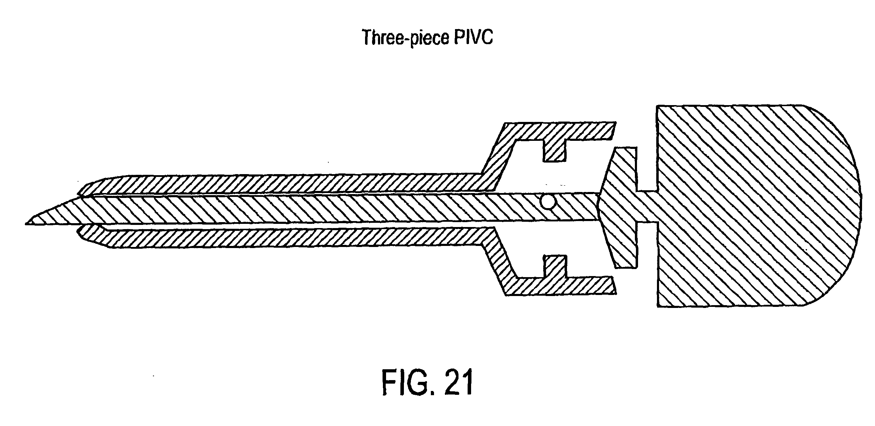

**Figures 19** and **20** illustrate another embodiment of a three-piece catheter device in accordance with one embodiment of the invention. **Figure 19** illustrates a two-piece needle, plug and head. Needle 840 may be hollow, or grooved and comprised of a polymeric compound, metal, or other suitable material. Optionally, a hole or an aperture 870 is located in needle 840 into hub compartment 950 of **Figure 20**. Hole or aperture 870 formed in needle 840 allows trapped gas to be released as fluid moves along the length of needle 840. Needle 840 is secured to a plug 850 and head 860, and this assembly is then inserted into the one-piece catheter hub 875 as shown in **Figure 21**. One-piece catheter and hub 875 comprises tube portion 900, hub portion 950 and supporting arms 960. Supporting arms 960 contact plug 850 after needle 840 is inserted into tube portion 900. Supporting arms 960 prevent plug 850 from moving distally as the healthcare worker inserts the catheter device into a patient.

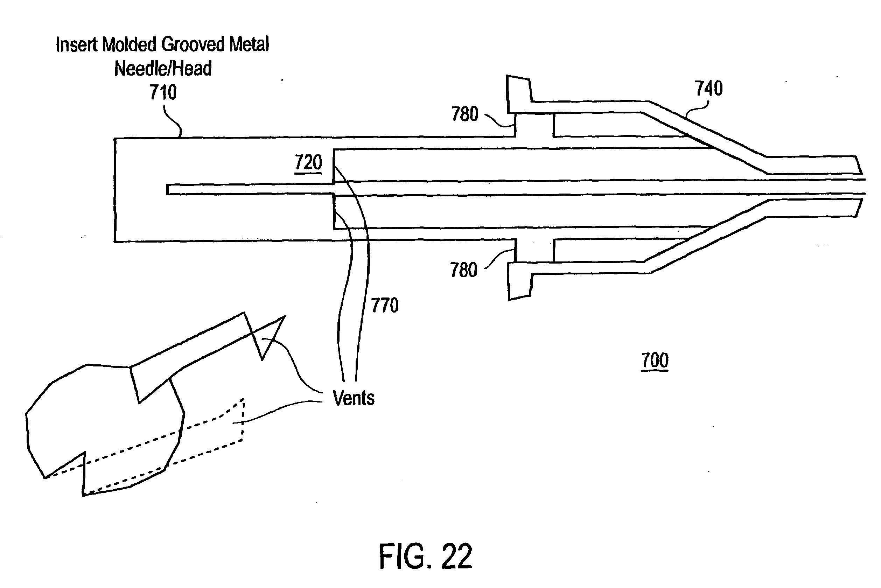

**Figure 22** illustrates an enlarged view of a three-piece catheter device 700 with vents in accordance with one embodiment of the invention. A grooved needle is then insert molded into a polymeric head 710 to form a tight connection 720 along the center length of the head. The grooved needle and head is then inserted into one-piece beveled catheter and hub 740. Hub 740 fits snugly over flanges 780 of head 710. Vents 770 are located at the proximal end of head 710. Vents 770 are formed by the connection of the metallic core pin with the metallic mold cavity during the insert molding process. Vents 770 allow trapped gas such as air to exit the catheter device to reduce pressure buildup within the catheter device as blood is drawn out of the vein.

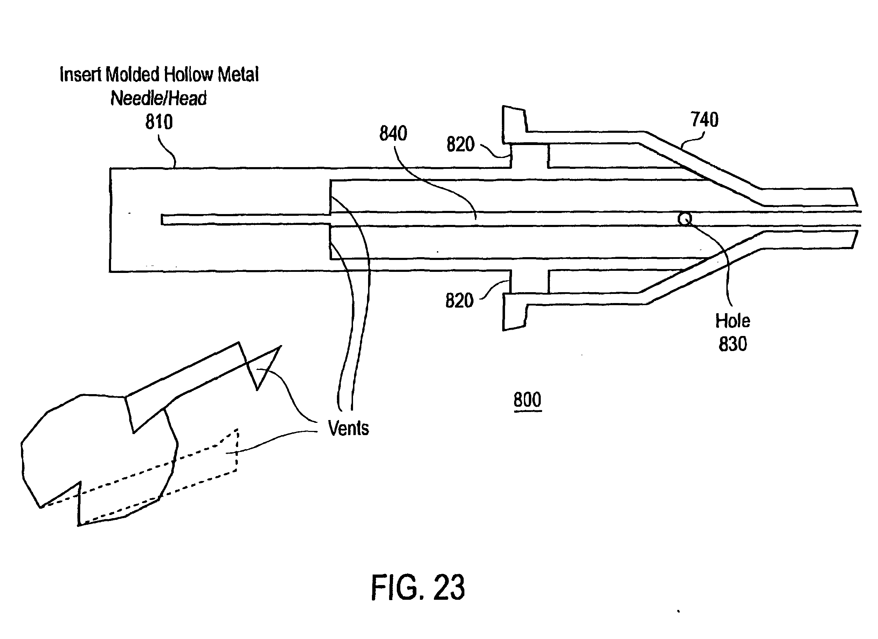

**Figure 23** illustrates a larger scale view of a three-piece catheter device 800 in accordance with one embodiment of the invention. A hollow needle is insert molded into a polymeric head 810 to form a tight connection along the center length of head 810. A hole or aperture 830 may be located within needle 840. Hole or aperture 830 extends through the upper portion of needle 840 but also could extend through the diametrically opposed wall of needle 840. The hollow needle and head is inserted into one-piece beveled catheter and hub 740. Hub 740 fits snugly over flanges 820 of head 810. Flanges 820 contact the inner surface of hub 740.

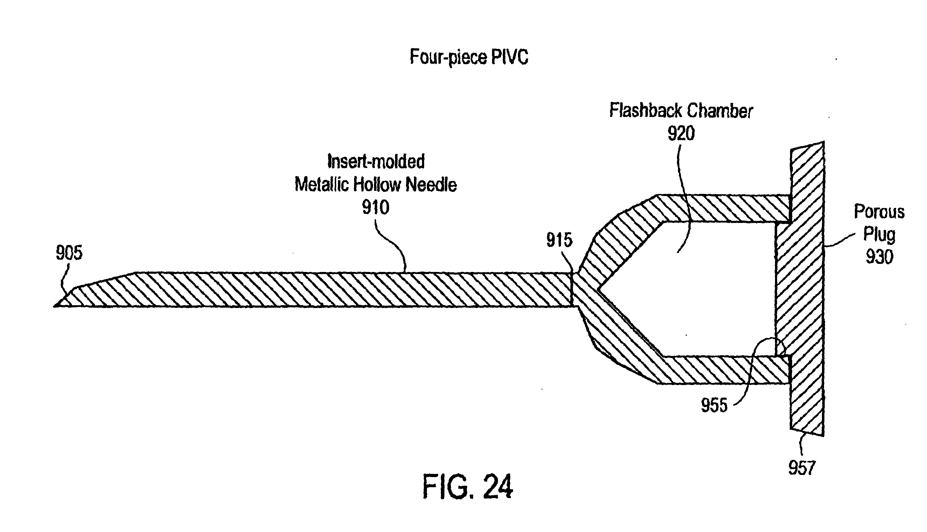

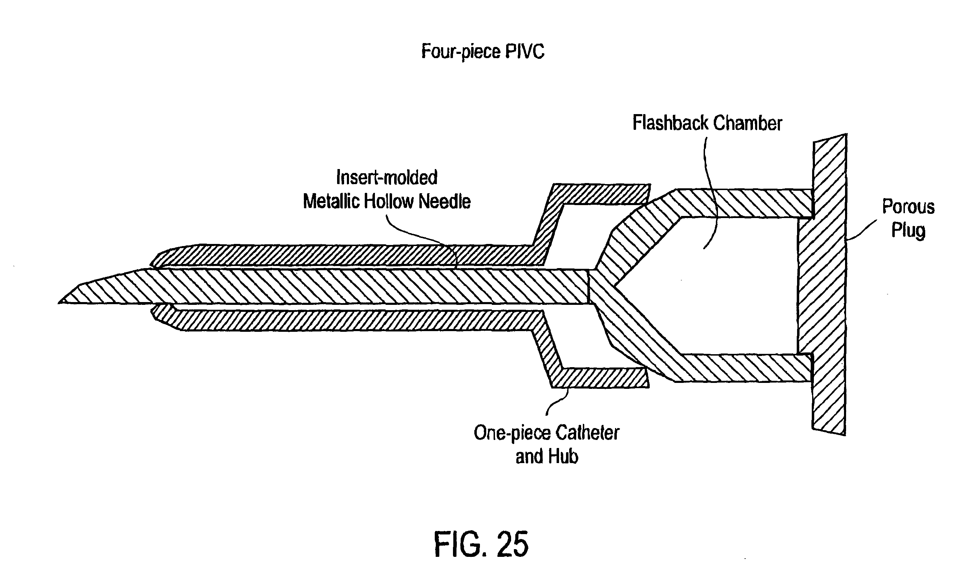

**Figures 24** and **25** illustrate a four-piece catheter device 900 assembled in accordance with one embodiment of the invention. In this embodiment of the invention. Needle, flashback chamber ("FBC") 920, and plug 930 comprise a three-piece assembly as shown in **Figure 24**. Needle 910 may be grooved or hollow. Needle 910 has a beveled distal tip 905 and a proximal end 915. The proximal end of needle 910 is secured to FBC 920 through insert molding. FBC 920 is then coupled to a porous plug 930 that fits within FBC 920. Porous plug 930 has an outer portion that extends over FBC 920 a certain length 955 and width 957 approximately in the range of 0.2-0.6 inches. Porous plug 930 allows ventilation of FBC upon needle and catheter insertion into the vein. This allows the blood to enter the FBC 920. The porous plug can also be removed upon demand to allow irrigation processes to occur using a syringe if necessary.

**Figure 25** illustrates a four-piece catheter device in accordance with one embodiment of the invention. Specifically, **Figure 25** illustrates three-piece needle, FBC, and plug 930 inserted into one-piece catheter and hub 170 of **Figure 2**. The assembly of the three-piece needle, FBC, and plug 930 may occur through the methods described herein or any conventional techniques.

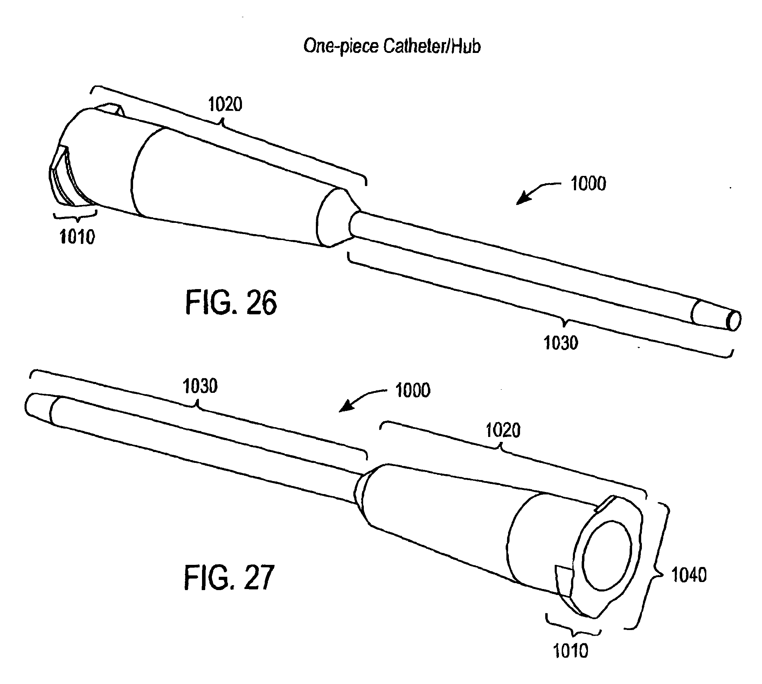

**Figures 26** and **27** illustrate a one-piece catheter and hub that may be used in a catheter device in accordance with one embodiment of the invention. Flange portion 1010 protrudes outwardly from hub portion 1020. Hub portion 1020 has an inner and outer diameter at the proximal end of hub portion 1020 that gradually decreases until hub portion 1020 ends at the distal portion of hub portion 1020. **Figure 27** further shows the hub portion having a top portion 1040 that has a substantially circular inner diameter and a substantially elliptical outer diameter. Catheter and hub 1000 also includes tube portion 1030.

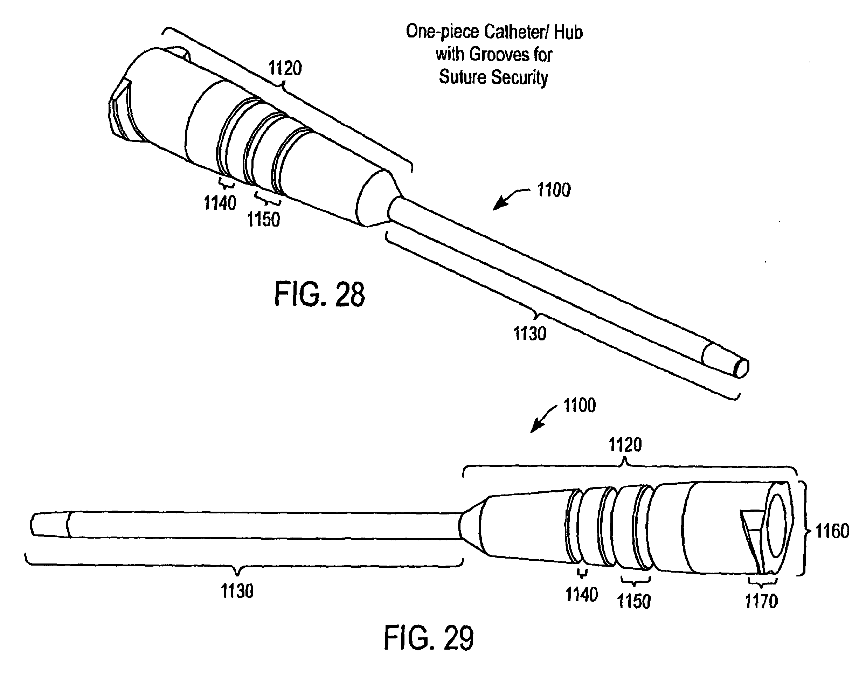

**Figures 28** through **31** illustrate a one-piece catheter and hub having grooves in hub portion 1120 in accordance with one embodiment of the invention. One-piece catheter and hub comprises a grooved hub portion 1120 and a tube portion 1130. Grooved hub portion 1120 has at least one groove 1140 and generally includes a plurality of grooves such as from 1 to 6 grooves. Grooves 1140 are separated by a length 1150 from another groove. Length 1150 is generally in the range of 0.1 inches to 0.8 inches. It will be appreciated that grooves 1140 may be evenly or unevenly spaced apart. Groove 1140 generally has a depth of 0.05 inches to 0.2 inches and a length of 0.1 inches to 0.3 inches.

Tube portion 1130 generally has a length of 0.5 inches to 3 inches. The diameter of the tube depends on the gauge to the catheter but may range from 0.01 inches to 0.1 inches. **Figure 29** further illustrates a top portion 1160 that shows one-piece catheter and hub 1100 having an inner diameter at the proximal end of hub portion 1120 that generally ranges from 0.05 inches to 0.35 inches and an outer diameter that generally ranges from 0.15 inches to 0.35 inches. The outer diameter also generally has a shape that is elliptical in nature. Flange portion is illustrated in 1170.

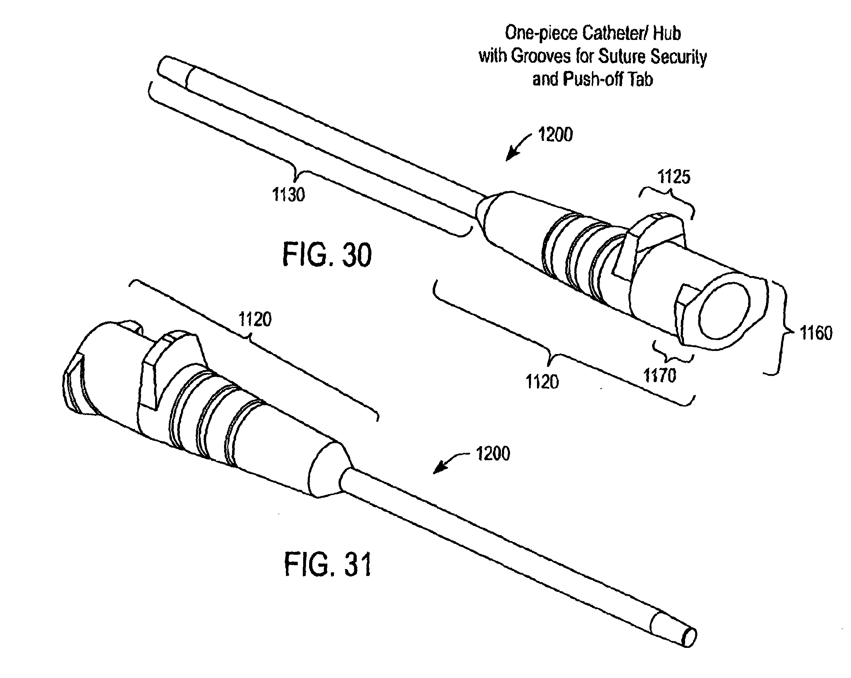

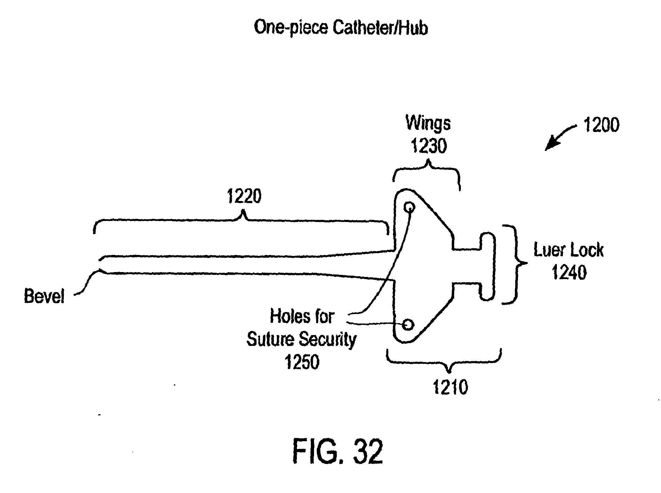

**Figures 30** and **31** illustrate a one-piece catheter and hub with a grooved hub portion 1120 with a push-off tab 1125 in accordance with one embodiment of the invention. **Figure 32** illustrates a one-piece catheter and hub 1200 in accordance with one embodiment of the invention. One-piece catheter and hub 1200 has a hub portion 1210 and a tube portion 1220. Hub portion 1210 comprises at least one wing 1230 and generally two wings. Hub portion 1210 also includes a luer lock 1240. Luer lock 1240 may be a male or female luer lock. Wings 1230 generally include holes or apertures 1250 for securing a suture.

In the preceding detailed description, the invention is described with reference to specific embodiments thereof. It will, however, be evident that various modifications and changes may be made thereto without departing from the broader spirit and scope of the invention as set forth in the claims. The specification and drawings are, accordingly, to be regarded in an illustrative rather than a restrictive sense.

## Claims

1. A catheter device comprising:
a needle and a head to the needle which are formed as a single integral piece;
wherein the needle and the head to the needle are inserted into a single integral piece catheter and hub.

2. The catheter device of claim 1, further comprising a plug located between the needle and head.

3. The catheter device of claim 1 or claim 2, wherein the head is a spiral head.

4. The catheter device of any one of claims 1 to 3, wherein the hub is a flashback chamber.

5. The catheter device of any one of claims 1 to 4, wherein the needle has at least one spiral or helical member which surrounds the needle.

6. The catheter device of any one of claims I to 5, wherein an aperture is located within the length of the needle.

7. The catheter device of any one of claims 1 to 6, wherein the head has at least one aperture for venting.

8. A catheter unit comprising:
a one-piece needle wherein a head to the needle is coupled to a one-piece catheter and hub.

9. The catheter unit of claim 8, wherein a plug is located between the needle and the head of the needle.

10. The catheter unit of claim 8 or claim 9, wherein the needle has a beveled distal tip.
